# EUROPEAN PATENT APPLICATION

(11) **EP 4 385 492 A1**
(43) Date of publication of application: **19.06.2024**
(21) Application number: 23188118.6
(22) Date of filing: 27.07.2023
(51) Int. Cl.: A61K 8/85, A61Q 5/02, A61Q 5/12, A61Q 19/10

(54) **COMPOSITION FOR APPLICATION TO A SURFACE OF KERATIN**

(30) Priority: 12.12.2022 US 202263431911 P
(71) Applicant: Kao USA, Inc., Cincinnati, OH 45214 (US)
(72) Inventor: JONES DAVIS, Amy R., Cincinnati, OH 45214 (US); LOWE, Robert M., Cincinnati, OH 45214 (US)
(74) Representative: Samson & Partner Patentanwälte mbB

(57) **Abstract**

A composition, for application to a surface of keratin, including a first polyesteramine, is disclosed. The first polyesteramine is a first conditioning agent present in an amount ranging from 0.1% to 99.9% by weight of the composition. The composition also includes a second polyesteramine. The second polyesteramine is a second conditioning agent present in an amount ranging from 0.1% to 99.9% by weight of the composition.

## Description

### FIELD OF TECHNOLOGY

The present application discloses a composition, and in particular, a composition for application to a surface of keratin.

### BACKGROUND

Hair care products for conditioning benefits work by making hair more hydrophobic via coating the hair. While many such products exist on the market, there is still room for improvement by giving a more hydrophobic surface coating on hair to provide conditioning benefits.

One solution is to use polyesteramines (or polyesters) in hair care products. Polyesteramines can be used, for instance, to enhance wettability or dispersion of such a product when used on hair.

However, even with the inclusion of polyesteramines, conventional cosmetic materials that impart conditioning and moisture enhancement to a surface of keratin (such as skin or hair) are often large sticky/tacky materials that can deposit unevenly on the surface. Over time, this causes build-up with repeated applications. This can result in a heavy weigh down and a dirty/greasy/coated feel, and often is only applicable to specific surfaces or types (e.g., coarse hair, damaged hair, and dry skin).

Even lower-weight materials not prone to build-up can produce a sticky/tacky feel, resulting in uneven deposition with poor spreadability. On hair, this can lead to reduced ease of combing and tangling with a sticky/tacky/gummy coated feel.

Furthermore, there is a push for cosmetic compositions, including hair care products, to remove ingredients such as silicones, sulfates, and parabens.

The disclosed methods and compositions address one or more of the aforementioned needs in the art.

### SUMMARY

Disclosed are compositions for application to a surface of keratin, for example, a surface of skin or a surface of hair. The composition may include a first polyesteramine as a first conditioning agent in an amount ranging from 0.1% to 99.9% by weight, and a second polyesteramine as a second conditioning agent in an amount ranging from 0.1% to 99.9% by weight.

In one aspect, the disclosed compositions may provide improved conditioning and moisturizing benefits (e.g., slippage, suppleness, softness, and alignment) across a surface of keratin, for instance, hair. Without intending to limit the claims by theory, it is believed that a combination of a first polyesteramine (for instance, polyester-11) and a second polyesteramine (for instance, polyester-37) contributes to improved conditioning and moisturizing benefits. Such a combination was found to have superior benefits in both a wet state and a dry state on a wide range of surfaces and textures. Additionally, in some aspects, the disclosed compositions may achieve such a feature while excluding ingredients such as silicones, sulfates, and parabens (i.e., containing 0% of such ingredients). The benefits imparted by the unique blend of polyesteramines were found to surpass those produced by conventional conditioning agents, or by each polyesteramine used independently. Furthermore, such a combination together with an additional conditioning agent is believed to further enhance these benefits.

### BRIEF DESCRIPTION OF THE DRAWINGS

This application file contains at least one drawing executed in color. Copies of this patent or patent application publication with color drawing(s) will be provided by the Office upon request and payment of the necessary fee.

Those of skill in the art will understand that the drawings, described below, are for illustrative purposes only. The drawings are not intended to limit the scope of the present teachings in any way.
FIG. 1 illustrates hair of a mannequin before and after treatment with an exemplary composition.
FIG. 2A illustrates hair of a panelist before treatment with an exemplary composition.
FIG. 2B illustrates hair of a panelist after treatment with an exemplary composition.
FIG. 2C illustrates hair of a panelist after treatment with an exemplary composition.
FIG. 3 illustrates hair of a panelist during and immediately after treatment with an exemplary composition.
FIG. 4A illustrates hair of a panelist before treatment with an exemplary composition.
FIG. 4B illustrates hair of a panelist after treatment on one side with an exemplary composition and on another side with a commercial standard.
FIG. 4C illustrates hair of a panelist after treatment with an exemplary composition.
FIG. 4D illustrates hair of a panelist after treatment with a commercial standard.
FIG. 5A illustrates hair of a panelist before treatment with an exemplary composition.
FIG. 5B illustrates hair of a panelist after treatment with an exemplary composition.
FIG. 5C illustrates hair of a panelist after treatment with an exemplary composition.
FIG. 6A illustrates a section of hair of a panelist before treatment with an exemplary composition.
FIG. 6B illustrates a section of hair of a panelist after treatment with an exemplary composition.
FIG. 6C illustrates a section of hair of a panelist after treatment with an exemplary composition.

### DETAILED DESCRIPTION

Unless otherwise noted, terms are to be understood according to conventional usage by those of ordinary skill in the relevant art. In case of conflict, the present document, including definitions, will control. Exemplary methods and materials are described below, although methods and materials similar or equivalent to those described herein may be used in practice or testing of the disclosed compositions and methods.

As used herein and in the appended claims, the singular forms "a," "and," "the", and "said" include plural referents unless the context clearly dictates otherwise. Thus, for example, reference to "a method" includes a plurality of such methods and reference to "a dose" includes reference to one or more doses and equivalents thereof known to those skilled in the art, and so forth.

A composition for application to a surface of keratin is presented. In certain versions, the composition may include a first polyesteramine and a second polyesteramine. Each polyesteramine may be present in an amount ranging from 0.1% to 99.9% by weight of the composition.

### Solvent

In one aspect, the disclosed composition may include a solvent. In one instance, the solvent is water or deionized water. In such an instance, the water may be present in an amount necessary to total a weight of the composition to 100% (balance), after inclusion of all other ingredients. The total amount of solvent typically ranges from 45% to 90%, or from 50% to 85%, or from 55% to 80%, or from 60% to 75%, or from 65% to 70% by weight of the composition. The total amount of solvent may range from 45%, or 50%, or 55%, or 60%, or 65% to 70%, or 75%, or 80%, or 85%, or 90% by weight of the composition. Water may serve as a medium for external phase oil-in-water emulsions and as a solvent for water-soluble materials.

### pH-adjuster

The composition may include at least one pH-adjuster for neutralizing a pH of the composition. Non-limiting examples of the at least one pH-adjuster include: sodium hydroxide, lactic acid, malic acid, succinic acid, and combinations thereof. The at least one pH-adjuster may be present in an anhydrous form or added into the composition with water as a carrier.

The at least one pH-adjuster may be present in an amount ranging from 0.00025% to 1%, or from 0.0005% to 0.75%, or from 0.001 % to 0.5%, or from 0.01% to 0.4%, or from 0.1 % to 0.3% by weight of the composition. The amount may range from 0.00025%, or 0.0005%, or 0.001 %, or 0.01%, or 0.1% to 0.3%, or 0.4%, or 0.5%, or 0.75%, or 1% by weight of the composition.

### Conditioning Agents

The composition may include at least one conditioning agent for enhancing sensory properties of the hair. Non-limiting examples of the at least one conditioning agent include: alkanes, fatty acids, triglycerides, silicones, esters, polyols, and cationic compounds. These may be, for instance, isopropyl palmitate, C₁₅₋₁₉ alkane, hydroxypropyl guar hydroxypropyltrimonium chloride, guar hydroxypropyltrimonium chloride, panthenol, polyquaternium-10, coconut oil, dimethyl isosorbide, propanediol, isopentyldiol, silicones, siloxanes, dimethicone, bis-cetearyl amodimethicone, behentrimonium chloride, cetrimonium chloride, dipropylene glycol, behenamidopropyl dimethylamine, and combinations thereof.

The at least one conditioning agent may be present in an amount ranging from 0.0025% to 5%, or from 0.005% to 4.5%, or from 0.0075% to 4%, or from 0.01% to 3.5%, or from 0.05% to 3%, or from 0.1% to 2.5%, or from 0.5% to 2%, or 0.75% to 1% by weight of the composition. The amount may also range from 0.0025%, or 0.005%, or 0.0075%, or 0.01%, or 0.05%, or 0.1%, 0.5%, or 0.75% to 1%, or 2%, or 2.5%, or 3%, or 3.5%, or 4%, or 4.5%, or 5% by weight of the composition.

The at least one conditioning agent may be an additional conditioning agent, that is in addition to polyesteramines, described below.

### Humectants

The composition may include at least one humectant for hydrating the hair. Non-limiting examples of the at least one humectant include erythritol and glycerin.

The at least one humectant may be present in an amount ranging from 0.1% to 6%, or from 0.5% to 5%, or from 1% to 4%, or from 2% to 3% by weight of the composition. The amount may range from 0.1%, or 0.5%, or 1%, or 2% to 3%, or 4%, or 5%, or 6% by weight of the composition.

### Solubilizer

The composition may include a solubilizer for thickening aqueous components of the composition. The solubilizer may be, for instance, sodium chloride. The solubilizer may be present in an amount ranging from 0.015% to 0.15%, or from 0.02% to 0.1%, or from 0.025% to 0.05% by weight of the composition. The amount may range from 0.015%, or 0.02%, or 0.025% to 0.05%, or 0.1%, or 0.15% by weight of the composition.

### Thickeners

The composition may include at least one thickener for providing emulsion stability to the composition. Non-limiting examples of thickeners include hydroxypropyl methylcellulose and hydroxyethylcellulose.

The at least one thickener may be present in an amount ranging from 0.075% to 3%, or from 0.1% to 2%, or from 0.5% to 1% by weight of the composition. The amount may range from 0.075%, or 0.1%, or 0.5% to 1%, or 2%, or 3% by weight of the composition.

### Opacifying Agent

The composition may include an opacifying agent for reducing transparency of the composition. The opacifying agent may be, for instance, glycol distearate.

The opacifying agent may be present in an amount ranging from 0.35% to 3%, or from 0.4% to 2.5%, or from 0.5% to 2%, or from 0.75% to 1.5% by weight of the composition. The amount may range from 0.35%, or 0.4%, or 0.5%, or 0.75% to 1.5%, or 2%, or 2.5%, or 3% by weight of the composition.

### Surfactants

The composition may include at least one surfactant. The at least one surfactant may contribute to wetting the hair and emulsifying impurities on the hair, as well as boosting foam/lather of the composition. The at least one surfactant may also boost foam formation of the composition. For the purpose of this application, the phrase "boost foam formation" refers to increasing surface viscosity of liquid surrounding individual bubbles in a foam. Non-limiting examples of the at least one surfactant include: cocamidopropyl betaine, coco-glucoside, decyl glucoside, sodium C₁₄₋₁₆ olefin sulfonate (may be 40% active), cocamide MEA, sodium laureth sulfate, sodium lauryl sulfate, and combinations thereof. The at least one surfactant may be present in an anhydrous form or added into the composition with water as a carrier.

The at least one surfactant may be present at an amount ranging from 0.2% to 25%, or from 0.5% to 20%, or from 1% to 15%, or from 2% to 10%, or from 4% to 8%, or from 5% to 7% by weight of the composition. The amount may range from 0.2%, or 0.5%, or 1%, or 2%, or 4%, or 5% to 7%, or 8%, or 10%, 15%, or 20%, or 25% by weight of the composition.

### Stabilizer

The composition may include at least one stabilizer for stabilizing emulsion formation of the composition. Non-limiting examples of the at least one stabilizer include: cetearyl alcohol, behenyl alcohol, cetyl alcohol, stearyl alcohol (for instance, National Formulary, or NF), and combinations thereof.

The at least one stabilizer may be present in an amount ranging from 0.2% to 15%, or from 0.25% to 10%, or from 0.5% to 8%, or from 1% to 7%, or from 2% to 6%, or from 3% to 5% by weight of the composition. The amount may range from 0.2%, or 0.25%, or 0.5%, or 1%, or 2%, or 3% to 5%, or 6%, or 7%, 8%, or 10%, or 15% by weight of the composition.

### Antimicrobial Agents

The composition may include at least one antimicrobial agent for disrupting a microbial cell when in contact with the composition. The microbial cell has a cell membrane encompassed by a cell wall. The at least one antimicrobial agent disrupts the microbial cell by interfering with the cell wall and the cell membrane. Non-limiting examples of the at least one antimicrobial agent include: methylchloroisothiazolinone, methylisothiazolinone, benzoic acid, benzyl alcohol, and combinations thereof. The at least one antimicrobial agent may be present in an anhydrous form or may be added into the composition with water as a carrier.

The at least one antimicrobial agent may be present in an amount ranging from 0.015% to 0.55%, or from 0.02% to 0.5%, or from 0.025% to 0.4%, or from 0.05% to 0.3%, or from 0.1% to 0.2% by weight of the composition, or as necessary to total the weight of the composition to 100% (QS). The amount may range from 0.015%, or 0.02%, or 0.025%, or 0.05%, or 0.1% to 0.2%, or 0.3%, or 0.4%, or 0.5%, or 0.55% by weight of the composition, or as necessary to total the weight of the composition to 100% (QS).

### Other Ingredients

The composition may further include other ingredients, having non-limiting functions such as: film-forming, hair fixative, binder, hair/skin conditioning, surfactant, humectant, viscosity increasing, viscosity decreasing, pH-adjusting, emulsion stabilizing, emollient, solvent, antimicrobial, chelating, anti-static. Non-limiting examples of such ingredients include: VP/VA copolymer, VP/methacrylamide/vinyl imidazole copolymer, propylene glycol, butylene glycol, amidoamine, isopropyl myristate, jojoba oil, argan oil, citric acid, ethanol, PVP, diheptyl succinate, capryloyl glycerin/sebacic acid copolymer, dicaprylyl carbonate, triheptanoin, acrylates/C₁₀₋₃₀ alkyl acrylate crosspolymer, polyquaternium-7, polyquaternium-11, polyquaternium-37, polyquaternium-39, PVP/DMAPA acrylates copolymer, brassicamidopropyl dimethylamine, stearamidopropyl dimethylamine, ethoxydiglycol, PPG-9, pentylene glycol, disodium EDTA, sodium lauroyl sarcosinate, sodium cocoyl isethionate, water (and) silicone quaternium-18 (and) trideceth-6 (and) trideceth-12, sodium laurylglucosides hydroxypropylsulfonate, sodium lauryl sulfoacetate, and disodium laureth sulfosuccinate .

Such ingredients may be present in an amount ranging from 0.005% to 60%, or from 0.01% to 55%, or from 0.1 % to 50%, or from 1 % to 40%, or from 5% to 30%, or from 10% to 20% by weight of the composition, or as necessary to total the weight of the composition to 100% (QS). The amount may range from 0.005%, or 0.01%, or 0.1%, or 1%, or 5%, or 10% to 20%, or 30%, or 40%, or 50%, or 55% by weight of the composition, or as necessary to total the weight of the composition to 100% (QS).

It should be understood that any of the above ingredients may be present in the composition but are not necessarily required for the interaction of the two polyesteramines, described below.

### Polyesteramines

Polyesteramines are generally defined in US Patent 7,101,538.

The composition may include a first polyesteramine. The first polyesteramine may have at least one ester linkage and at least one monofunctional carboxylic acid group. Non-limiting examples of carboxylic acid groups include: benzoic acid, 2-ethylhexanoic acid, nonanoic acid, lauric acid, myristic acid, palmitic acid, myristic acid, palmitic acid, stearic acid, isostearic acid, coconut fatty acid, oleic acid, behenic acid, and/or derivatives thereof. The first polyesteramine may also have at least one additional carboxylic acid group, including (but not limited to): adipic acid, cyclohexanedicarboxylic acid, sebacic acid, azelaic acid, dodecanedioic acid, phthalic acid, isophthalic acid, terephthalic acid, trimellitic acid, dimer acid, trimer acid, 2,6-naphthalene dicarboxylic acid, pyromellitic acid, and/or derivatives thereof.

A non-limiting example of the first polyesteramine is polyester-11 (for instance, Kerazyne^{™} MB from Inolex, INCI Monograph ID 22222; CAS No. 905598-82-5) as a first conditioning agent. Polyester-11 is a copolymer of adipic acid and methyl diethanolamine crosslinked with glycerin and terminated with coconut acid. Polyester-11 may be present in an amount ranging from 0.1% to 99.9%, or from 0.25% to 80%, or from 0.3% to 70%, or from 0.4% to 60%, or from 0.5% to 50%, or from 0.75% to 40%, or from 1% to 25%, or from 1.5% to 10%, or from 2% to 5% by weight of the composition. The amount may also range from 0.1%, or 0.25%, or 0.3%, or 0.4%, or 0.5%, or 0.75%, or 1%, or 1.5%, or 2% to 5%, or 10%, or 25%, or 40%, or 50%, or 60%, or 70%, or 80%, or 99.9% by weight of the composition.

The composition may also include a second polyesteramine. The second polyesteramine may have at least one ester linkage and at least one monofunctional carboxylic acid group. Non-limiting examples of carboxylic acid groups include: benzoic acid, 2-ethylhexanoic acid, nonanoic acid, lauric acid, myristic acid, palmitic acid, myristic acid, palmitic acid, stearic acid, isostearic acid, coconut fatty acid, oleic acid, behenic acid, and/or derivatives thereof. The second polyesteramine may also have at least one additional carboxylic acid group, including (but not limited to): adipic acid, cyclohexanedicarboxylic acid, sebacic acid, azelaic acid, dodecanedioic acid, phthalic acid, isophthalic acid, terephthalic acid, trimellitic acid, dimer acid, trimer acid, 2,6-naphthalene dicarboxylic acid, pyromellitic acid, and/or derivatives thereof.

A non-limiting example of the second polyesteramine is polyester-37 (for instance, Clarisilk^{™} by Inolex, INCI Monograph ID 32935; CAS No. 896732-68-6) as a second conditioning agent. Polyester-37 is a copolymer of adipic acid and methyl diethanolamine crosslinked with glycerin and terminated with isostearic acid. Polyester-37 may be present in an amount ranging from 0.1% to 99.9%, or from 0.25% to 80%, or from 0.3% to 70%, or from 0.4% to 60%, or from 0.5% to 50%, or from 0.75% to 40%, or from 1% to 25%, or from 1.5% to 10%, or from 2% to 5% by weight of the composition. The amount may also range from 0.1%, or 0.25%, or 0.3%, or 0.4%, or 0.5%, or 0.75%, or 1%, or 1.5%, or 2% to 5%, or 10%, or 25%, or 40%, or 50%, or 60%, or 70%, or 80%, or 99.9% by weight of the composition.

In one aspect, the two polyesteramines may have different viscosities. For example, the first polyesteramine may have a viscosity ranging from 200 cp to 2000 cp, or from 300 cp to 1800 cp, or from 400 cp to 1600 cp, or from 500 cp to 1500 cp, or from 600 cp to 1300 cp, or from 700 cp to 1200 cp. In one aspect, the first polyesteramine may be polyester-11, and may have a viscosity ranging from 200 cp, or 300 cp, or 400 cp, or 500 cp, or 600 cp, or 700 cp to 1200 cp, or 1300 cp, or 1500 cp, or 1600 cp, or 1800 cp, or 2000 cp. The second polyesteramine may have a viscosity ranging from 2200 cp to 7500 cp, or from 2300 cp to 7000 cp, or from 2400 cp to 6500 cp, or from 2500 cp to 6000 cp, or from 2700 cp to 5500 cp or, from 3000 cp to 5000 cp. In one aspect, the second polyesteramine may be polyester-37, and may have a viscosity ranging from 2200 cp, or 2300 cp, or 2400 cp, or 2500 cp, or 2700 cp, or 3000 cp to 5000 cp, or 5500 cp, or 6000 cp, or 6500 cp, or 7000 cp, or 7500 cp. The viscosities mentioned herein are Brookfield viscosities measured at 25°C.

In one instance, the first polyesteramine and the second polyesteramine (for example, polyester-11 to polyester-37) may be present in a ratio ranging from 1:999 to 999:1, or from 1:320 to 320:1, or from 3:700 to 700:3, or from 1:150 to 150:1, or from 1:100 to 100:1, or from 3:160 to 160:3, or from 1:25 to 25:1, or from 3:20 to 20:3, or from 2:5 to 5:2. In other instances, the ratio may be 10:1, 1:2, 3:1, 2:1, 1:17, 17:1, or 1:10. In one instance, the ratio is 1:1.

The composition may include a combined amount of the first polyesteramine (for instance, polyester-11) and the second polyesteramine (for instance, polyester-37) ranging from 0.1% to 100%, or from 0.2% to 95% or from 0.5% to 90%, or from 1% to 85%, or from 5% to 80%, or from 10% to 75%, or from 15% to 70%, or from 20% to 65%, or from 25% to 60%, or from 30% to 55%, or from 40% to 50% by weight of the composition. The combined amount may range from 0.1%, or 0.2%, or 0.5%, or 1%, or 5%, or 10%, or 15%, or 20%, or 25%, or 30%, or 40% to 50%, or 55%, or 60%, or 65%, or 70%, or 75%, or 80%, or 85%, or 90%, or 95%, or 100% by weight of the composition.

Polyester-11 and polyester-37 may both be present with the at least one additional conditioning agent, described above, to further enhance at least one sensory feature of the hair.

While not intending to limit the scope of the claims by theory, it is believed that the blend of at least two polyesteramines (for instance, polyester-11 and polyester-37) results in a composition that can be evenly distributed across a surface of keratin, such as hair. It is further believed that such a blend in combination with at least one additional conditioning agent (such as the conditioning agents described above) results in a composition that can be evenly distributed across a surface of keratin, such as hair. This allows for benefits such as (but not limited to): improved hair alignment, reduced tangling, reduced hair breakage during combing, increased softness and smoothness of the hair. The combination may also provide increased slippage of water across the hair, for instance, when rinsing off the composition after use, such that the hair fibers slip apart, resulting in a silky-smooth appearance.

### Silicones, Sulfates, and Parabens

In one aspect, the composition may include less than 0.1%, or less than 0.05%, or less than 0.01% by weight of at least one of: silicones, sulfates, and parabens. In another version, the composition may include less than 0.1%, or less than 0.05%, or less than 0.01% by weight of all such ingredients. In another aspect, the composition may free of (i.e., contains 0% by weight) at least one of: silicones, sulfates, and parabens. In another aspect, the composition may be free of all such ingredients.

### Examples

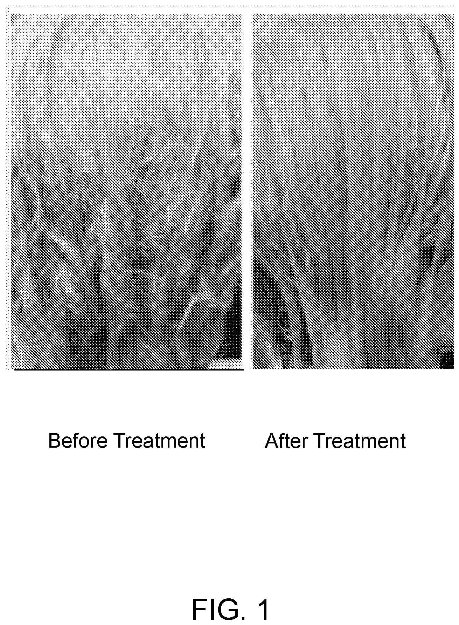

FIG. 1: Exemplary compositions were applied to hair of a mannequin. Photos were taken before and after application of the exemplary compositions. Before application, the hair was treated with a commercial standard shampoo (amounts per discretion of stylist). The commercial standard did not contain the combination of polyesteramines. The hair was found to be tangled and could not be combed through. The hair was then treated with an exemplary booster treatment added to a commercial conditioner (amounts per discretion of stylist). After treatment, the hair was found to be detangled as a result of the exemplary composition and easily combed through.

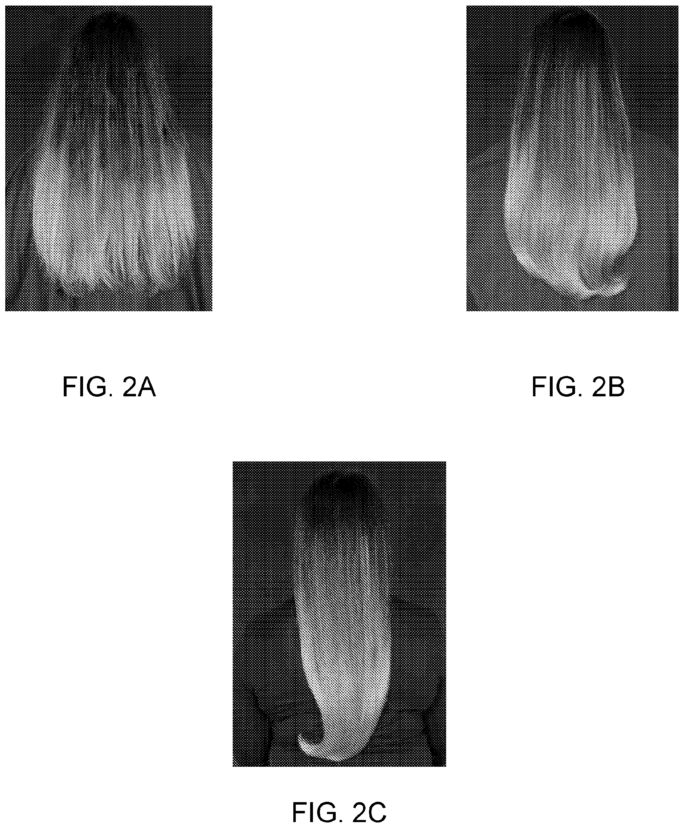

FIGS. 2A-3: Hair of the first panelist was first treated with 11g of a commercial standard shampoo and 13.2g of a commercial standard conditioner (amounts per discretion of stylist). The commercial standard did not contain the combination of polyesteramines. After treatment, the hair was blow-dried smooth with a round brush. The hair was found to be tangled (FIG. 2A). The following day, the hair was treated with 10.8g of an exemplary shampoo and 15.7g of an exemplary conditioner (amounts per discretion of stylist). After treatment, the hair was blow-dried smooth with a round brush. The hair was found to be detangled as a result of the exemplary compositions (FIG. 2B). The panelist then conducted one wash at home using their typical shampoo and conditioner. Then, a week after FIG. 2B was taken, the hair was treated with 15g of the exemplary shampoo and 30g of an exemplary mask (amounts per discretion of stylist). After treatment, the hair was blow-dried smooth with a round brush. The hair was found to be detangled as a result of the exemplary compositions (FIG. 2C). Photos were taken with a black background with camera set to f18 aperture. Exemplary compositions did not contain silicone.

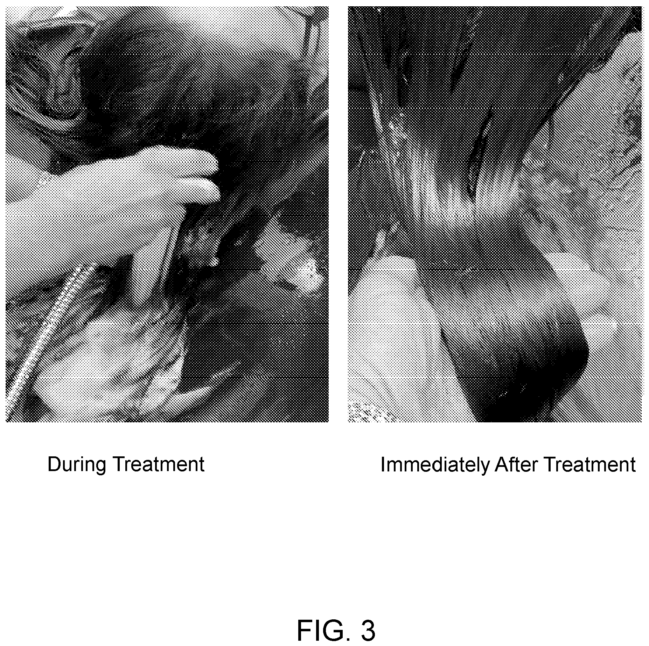

FIG. 3 illustrates the hair detangling benefits after application of the exemplary conditioner when rinsing, and immediately after in the wet state without combing or brushing the hair. The hair was found to be detangled immediately after application of the exemplary compositions.

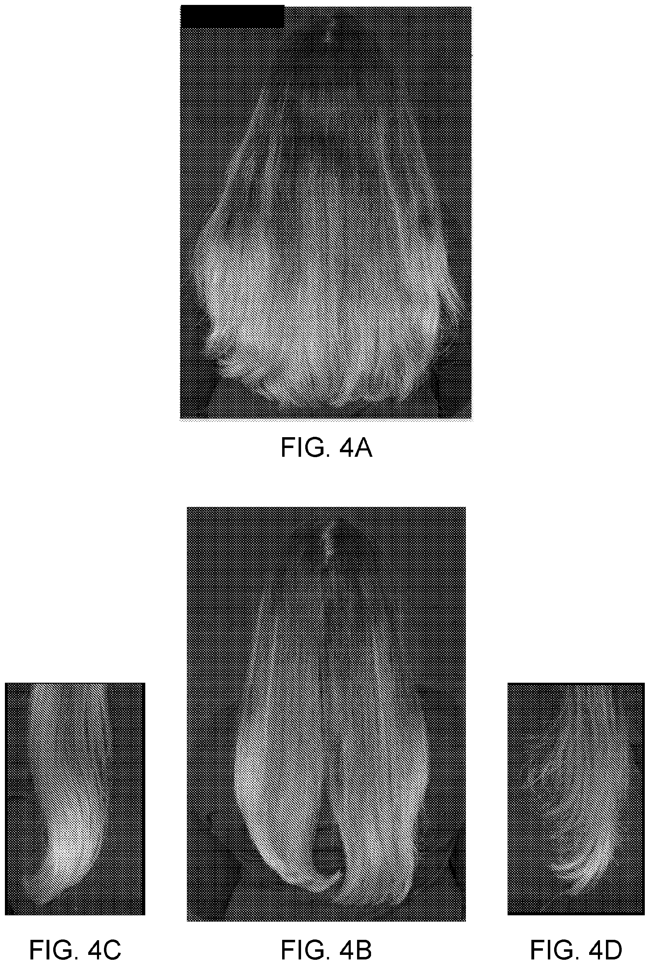

FIGS. 4A-4D: Hair of the first panelist was treated on the left side with exemplary compositions and on the right side with a commercial standard that did not contain the combination of polyesteramines but did contain silicone. Before treatment, the first panelist washed their hair with their typical shampoo and conditioner, and prior to pictures once at the salon the hair was blow-dried smooth with a round brush (FIG. 4A). On the left side, the hair was treated with 6.3g of the exemplary shampoo and 8.4g of the exemplary conditioner (amounts per discretion of stylist). On the right side, the hair was treated with 6.8g of commercial standard shampoo and 8.4g of commercial standard conditioner (amounts per discretion of stylist). FIGS. 4B-4D illustrate the comparison between treatment types. The exemplary compositions resulted in greater detangling, alignment, and smoothness of the hair. Photos were taken with a black background with camera set to f18 aperture. Exemplary compositions did not contain silicone.

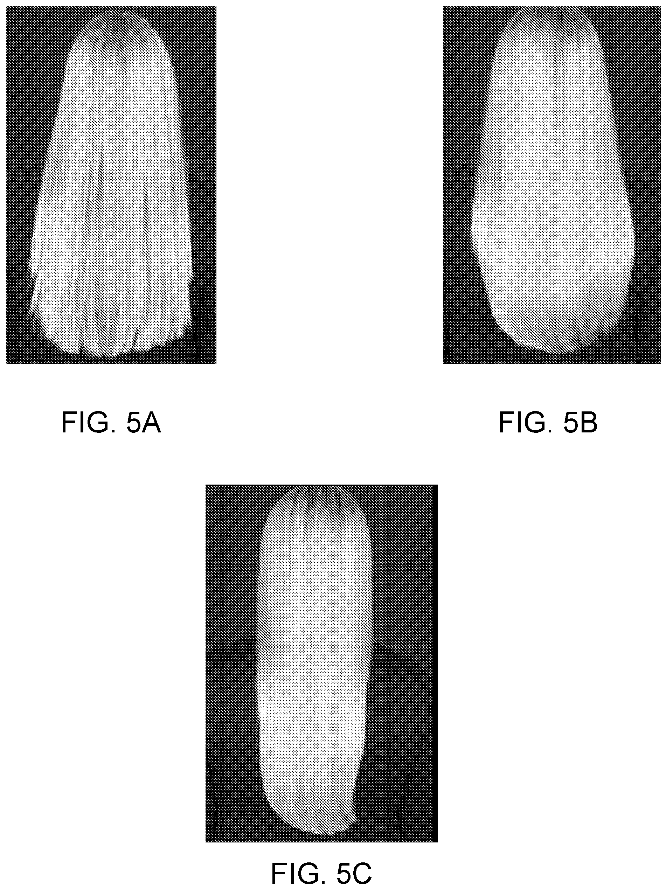

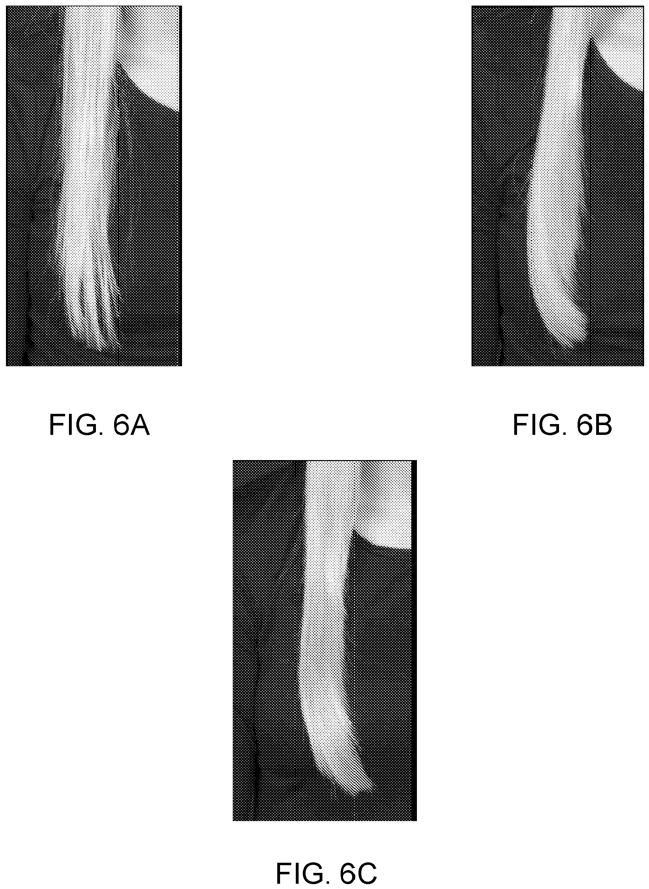

FIGS. 5A-6C: Exemplary compositions were applied to hair of a second panelist. Photos were taken before and after application of the exemplary compositions. Before application, the panelist washed their hair with their typical shampoo and conditioner, and prior to pictures once at the salon the hair was blow-dried smooth with a round brush (FIGS. 5A and 6A). The hair was then treated with 11g of the exemplary shampoo and 12.5g of the exemplary conditioner (amounts per discretion of stylist). After treatment, the hair was blow-dried smooth with a round brush. FIGS. 5B and 6B illustrate the hair detangled after treatment. Two days later, the hair was treated with 13.5g of the exemplary shampoo and 22g of the exemplary mask (amounts per discretion of stylist). After treatment, the hair was blow-dried smooth with a round brush. FIGS. 5C and 6C illustrate the hair detangled after treatment. Photos were taken with a black background with camera set to f18 aperture. Exemplary compositions did not contain silicone.

### Exemplary Compositions

Below are non-limiting examples of the composition and non-limiting methods of making such compositions.

### Shampoo/Bodywash Compositions

Example 1: water (balance), malic acid (1%), lactic acid (0.2%), sodium laureth sulfate (15%), coco-glucoside (5%), cocamidopropyl betaine (10%), polyester-11 (1%), polyester-37 (1%), alkane (1%), polyquaternium-39 (0.5%), polyquaternium-10 (0.2%), guar hydroxypropyltrimonium chloride (0.2%), PPG-9 (0.01%), capryloyl glycerin/sebacic acid copolymer (0.27%), cetyl alcohol (1.5%), cocamide MEA (1%), glycol distearate (2%), dimethyl isosorbide (0.25%), propanediol (0.5%), isopentyldiol (0.1%), diheptyl succinate (0.23%), benzyl alcohol (0.25%), additional antimicrobial agent/s (QS), fragrance (QS). Combined amount of polyester-11 and polyester-37: 2%.

Example 2: water (balance), citric acid (0.75%), lactic acid (0.5%), sodium laureth sulfate (15%), sodium lauryl sulfate (10%), cocamidopropyl betaine (5%), decyl glucoside (2%), polyester-11 (0.25%), polyester-37 (0.75%), coconut oil (1%), polyquaternium-10 (0.6%), dimethicone (0.3%), bis-cetearyl amodimethicone (0.5%), PPG-9 (0.01%), dimethyl isosorbide (0.5%), isopentyldiol (0.75%), propylene glycol (0.5%), benzyl alcohol (0.25%), additional antimicrobial agent/s (QS), fragrance (QS). Combined amount of polyester-11 and polyester-37: 1%.

Example 3: water (balance), malic acid (1%), sodium laureth sulfate (16%), cocamidopropyl betaine (10%), decyl glucoside (4%), polyester-11 (0.5%), polyester-37 (0.5%), jojoba oil (0.25%), argan oil (0.25%), polyquaternium-7 (2%), polyquaternium-10 (0.3%), hydroxypropyl methylcellulose (0.2%), dimethicone (0.75%), PPG-9 (0.01%), water (and) silicone quaternium-18 (and) trideceth-6 (and) trideceth-12 (0.5%), dimethyl isosorbide (0.8%), isopentyldiol (0.5%), pentylene glycol (0.5%), additional antimicrobial agent/s (QS), fragrance (QS). Combined amount of polyester-11 and polyester-37: 1%.

Example 4: water (balance), succinic acid (0.5%), citric acid (0.5%), sodium laureth sulfate (16%), sodium lauryl sulfate (9%), coco-glucoside (7.5%), cocamidopropyl betaine (7.5%), polyester-11 (2%), polyester-37 (0.5%), coconut oil (0.5%), alkane (1%), polyquaternium-7 (2%), polyquaternium-10 (0.25%), guar hydroxypropyltrimonium chloride (0.1%), PPG-9 (0.01%), cetyl alcohol (0.5%), cocamide MEA (1%), glycol distearate (1.5%), dimethyl isosorbide (0.75%), isopentyldiol (0.25%), propylene glycol (1%), pentylene glycol (0.5%), additional antimicrobial agent/s (QS), fragrance (QS). Combined amount of polyester-11 and polyester-37: 2.5%.

Example 5: water (balance), malic acid (0.5%), citric acid (0.5%), coco-glucoside (5%), cocamidopropyl betaine (10%), decyl glucoside (5%), sodium C₁₄₋₁₆ olefin sulfonate 40% (10%), polyester-11 (1%), polyester-37 (0.1%), dicaprylyl carbonate (1%), polyquaternium-7 (1.5%), polyquaternium-10 (0.2%), hydroxypropyl methylcellulose (0.2%), guar hydroxypropyltrimonium chloride (0.2%), cocamide MEA (1%), glycol distearate (2%), dimethyl isosorbide (0.25%), propanediol (0.5%), isopentyldiol (0.1%), benzyl alcohol (0.25%), additional antimicrobial agent/s (QS), fragrance (QS). Combined amount of polyester-11 and polyester-37: 1.1%.

Example 6: water (balance), succinic acid (0.75%), lactic acid (0.15%), sodium lauroyl sarcosinate (2%), sodium cocoyl isethionate (2%), coco-glucoside (4.5%), cocamidopropyl betaine (9%), decyl glucoside (4%), polyester-11 (0.1%), polyester-37 (0.5%), alkane (0.25%), jojoba oil (0.15%), polyquaternium-7 (1%), polyquaternium-10 (0.2%), hydroxypropyl methylcellulose (0.6%), guar hydroxypropyltrimonium chloride (0.1%), dimethicone (0.3%), bis-cetearyl amodimethicone (0.5%), cetyl alcohol (0.5%), cocamide MEA (1%), glycol distearate (2%), dimethyl isosorbide (0.5%), isopentyldiol (0.75%), propylene glycol (0.5%), benzyl alcohol (0.25%), additional antimicrobial agent/s (QS), fragrance (QS). Combined amount of polyester-11 and polyester-37: 0.6%.

Example 7: water (balance), lactic acid (0.8%), sodium laurylglucosides hydroxypropylsulfonate (9%), coco-glucoside (4%), cocamidopropyl betaine (2%), decyl glucoside (4%), polyester-11 (1%), polyester-37 (0.5%), coconut oil (1%), argan oil (0.5%), polyquaternium-7 (1.5%), polyquaternium-10 (0.2%), hydroxypropyl methylcellulose (0.2%), guar hydroxypropyltrimonium chloride (0.2%), dimethicone (0.75%), water (and) silicone quaternium-18 (and) trideceth-6 (and) trideceth-12 (0.5%), dimethyl isosorbide (0.8%), isopentyldiol (0.5%), pentylene glycol (0.5%), benzyl alcohol (0.25%), additional antimicrobial agent/s (QS), fragrance (QS). Combined amount of polyester-11 and polyester-37: 1.5%.

Example 8: water (balance), malic acid (0.9%), sodium lauroyl sarcosinate (2%), sodium cocoyl isethionate (2%), coco-glucoside (4.5%), cocamidopropyl betaine (2%), decyl glucoside (4.5%), polyester-11 (1%), polyester-37 (2%), alkane (1%), polyquaternium-39 (0.5%), polyquaternium-10 (0.3%), hydroxypropyl methylcellulose (0.6%), guar hydroxypropyltrimonium chloride (0.15%), capryloyl glycerin/sebacic acid copolymer (0.41%), cocamide MEA (1%), glycol distearate (2%), dimethyl isosorbide (0.75%), isopentyldiol (0.25%), propylene glycol (1%), pentylene glycol (0.5%), diheptyl succinate (0.34%), benzyl alcohol (0.25%), additional antimicrobial agent/s (QS), fragrance (QS). Combined amount of polyester-11 and polyester-37: 3%.

Add ingredients into a first mixing vessel in the order shown (minus waxes, fatty alcohols, additional antimicrobial agent/s, and fragrance), and heat to 75°C-85°C, forming a first mixture. Mix after each ingredient addition to ensure solubility and homogeneity, before adding a subsequent ingredient.

In a second mixing vessel, pre-melt and mix waxes and fatty alcohols, heating to 75°C-85°C, forming a second mixture. Once melted and homogeneous, add second mixture to first mixture (at 75°C-85°C) to form a third mixture, mixing until homogeneous.

Begin cooling third mixture to 40°C before adding additional antimicrobial agent/s and fragrance to form a final composition.

### Conditioner Compositions

Example 9: water (balance), malic acid (1%), cetearyl alcohol (5%), cetrimonium chloride (1%), behentrimonium chloride (3%), polyester-11 (1%), polyester-37 (1%), alkane (1%), isopropyl palmitate (0.75%), benzyl alcohol (0.5%), diheptyl succinate (0.23%), capryloyl glycerin/sebacic acid copolymer (0.27%), propylene glycol (1%), additional antimicrobial agent/s (QS), fragrance (QS). Combined amount of polyester-11 and polyester-37: 2%.

Example 10: water (balance), lactic acid (0.75%), behenyl alcohol (1%), cetearyl alcohol (4.5%), behenamidopropyl dimethylamine (1.6%), behentrimonium chloride (1.4%), polyester-11 (2%), polyester-37 (3%), coconut oil (1%), isopropyl myristate (1.5%), dimethicone (0.3%), bis-cetearyl amodimethicone (0.5%), benzyl alcohol (0.5%), additional antimicrobial agent/s (QS), fragrance (QS). Combined amount of polyester-11 and polyester-37: 5%.

Example 11: water (balance), succinic acid (0.8%), behenyl alcohol (1%), cetyl alcohol (2%), stearyl alcohol NF (3%), brassicamidopropyl dimethylamine (3%), behentrimonium chloride (0.5%), polyester-11 (0.5%), polyester-37 (0.5%), jojoba oil (0.25%), argan oil (0.25%), isopropyl palmitate (0.25%), dimethicone (0.75%), water (and) silicone quaternium-18 (and) trideceth-6 (and) trideceth-12 (0.5%), propylene glycol (0.5%), additional antimicrobial agent/s (QS), fragrance (QS). Combined amount of polyester-11 and polyester-37: 1%.

Example 12: water (balance), malic acid (0.2%), citric acid (0.6%), behenyl alcohol (2%), cetyl alcohol (4.5%), stearyl alcohol NF (0.5%), stearamidopropyl dimethylamine (2.5%), cetrimonium chloride (0.5%), polyester-11 (3%), polyester-37 (1%), coconut oil (0.5%), alkane (1%), isopropyl myristate (0.5%), propylene glycol (0.5%), ethoxydiglycol (0.5%), additional antimicrobial agent/s (QS), fragrance (QS). Combined amount of polyester-11 and polyester-37: 4%.

Example 13: water (balance), malic acid (1%), cetearyl alcohol (5%), cetrimonium chloride (1%), behentrimonium chloride (3%), polyester-11 (0.5%), polyester-37 (1%), isopropyl palmitate (2%), dicaprylyl carbonate (1%), benzyl alcohol (0.5%), propylene glycol (1%), additional antimicrobial agent/s (QS), fragrance (QS). Combined amount of polyester-11 and polyester-37: 1.5%.

Example 14: water (balance), lactic acid (0.75%), behenyl alcohol (1%), cetearyl alcohol (4.5%), behenamidopropyl dimethylamine (1.6%), behentrimonium chloride (1.4%), polyester-11 (0.2%), polyester-37 (1%), alkane (0.25%), jojoba oil (0.15%), isopropyl palmitate (1%), dimethicone (0.3%), bis-cetearyl amodimethicone (0.5%), benzyl alcohol (0.5%), additional antimicrobial agent/s (QS), fragrance (QS). Combined amount of polyester-11 and polyester-37: 1.2%.

Example 15: water (balance), succinic acid (0.8%), behenyl alcohol (1%), cetyl alcohol (2%), stearyl alcohol NF (3%), brassicamidopropyl dimethylamine (3%), behentrimonium chloride (0.5%), polyester-11 (3%), polyester-37 (0.3%), coconut oil (1%), argan oil (0.5%), isopropyl myristate (2%), dimethicone (0.75%), water (and) silicone quaternium-18 (and) trideceth-6 (and) trideceth-12 (0.5%), propylene glycol (0.5%), additional antimicrobial agent/s (QS), fragrance (QS). Combined amount of polyester-11 and polyester-37: 3.3%.

Example 16: water (balance), malic acid (0.02%), lactic acid (0.6%), behenyl alcohol (1%), cetyl alcohol (0.5%), stearyl alcohol NF (4.5%), behenamidopropyl dimethylamine (3%), polyester-11 (2%), polyester-37 (1%), alkane (1%), isopropyl myristate (1%), diheptyl succinate (0.34%), capryloyl glycerin/sebacic acid copolymer (0.41%), additional antimicrobial agent/s (QS), fragrance (QS). Combined amount of polyester-11 and polyester-37: 3%.

Add ingredients into a first mixing vessel in the order shown (minus waxes, fatty alcohols, silicone materials, additional antimicrobial agent/s, and fragrance), and heat to 75°C-85°C, forming a first mixture. Mix after each ingredient addition to ensure solubility and homogeneity, before adding a subsequent ingredient.

In a second mixing vessel, pre-melt and mix waxes and fatty alcohols, heating to 75°C-85°C, forming a second mixture. Once melted and homogeneous, add second mixture to first mixture (at 75°C-85°C) to form a third mixture, mixing until homogeneous.

Begin cooling third mixture to 55°C. At 55°C, add silicone materials to third mixture to form a fourth mixture. Mix until homogeneous. Continue cooling to 51°C-53°C, then homogenize.

Continue cooling fourth mixture to 40°C before adding additional antimicrobial agent/s and fragrance to form a final composition.

### Serum/Booster Compositions

Example 17: water (balance), cetrimonium chloride (1%), guar hydroxypropyltrimonium chloride (0.1%), triheptanoin (2%), bis-cetearyl amodimethicone (1%), cetearyl alcohol (3%), cetyl alcohol (1%), polyester-11 (1%), polyester-37 (1%), glycerin (3%), alkane (1%), isopropyl palmitate (0.75%), dimethyl isosorbide (0.16%), propanediol (0.24%), citric acid (QS%), additional antimicrobial agent/s (QS), fragrance (QS). Combined amount of polyester-11 and polyester-37: 2%.

Example 18: water (balance), polyquaternium-11 (2%), amidoamine (0.5%), guar hydroxypropyltrimonium chloride (0.2%), polyquaternium-37 (0.75%), triheptanoin (4%), bis-cetearyl amodimethicone (2%), water (and) silicone quaternium-18 (and) trideceth-6 (and) trideceth-12 (0.01%), dimethicone (5%), panthenol (0.25%), stearyl alcohol (2.5%), cetyl alcohol (0.5%), polyester-11 (3%), polyester-37 (3%), coconut oil (1%), glycerin (0.5%), isopropyl myristate (1.5%), dicaprylyl carbonate (0.2%), propanediol (0.75%), isopentyldiol (0.5%), citric acid (QS%), additional antimicrobial agent/s (QS), fragrance (QS). Combined amount of polyester-11 and polyester-37: 6%.

Example 19: water (balance), cetrimonium chloride (0.5%), amidoamine (1%), polyquaternium-37 (1%), diheptyl succinate (0.23%), capryloyl glycerin/sebacic acid copolymer (0.27%), water (and) silicone quaternium-18 (and) trideceth-6 (and) trideceth-12 (0.25%), dimethicone (0.5%), panthenol (0.1%), stearyl alcohol (0.5%), cetearyl alcohol (3%), polyester-11 (0.2%), polyester-37 (2%), glycerin (6%), jojoba oil (0.25%), argan oil (0.25%), isopropyl palmitate (0.25%), propylene glycol (0.5%), isopentyldiol (0.5%), citric acid (QS), additional antimicrobial agent/s (QS), fragrance (QS). Combined amount of polyester-11 and polyester-37: 2.2%.

Example 20: water (balance), siloxane (35%), dimethicone (10%), polyester-11 (35%), polyester-37 (10%), coconut oil (0.5%), glycerin (2%), alkane (1%), isopropyl myristate (0.5%), propanediol (1%), citric acid (QS), additional antimicrobial agent/s (QS), fragrance (QS). Combined amount of polyester-11 and polyester-37: 45%.

Example 21: water (balance), siloxane (35%), dimethicone (10%), polyester-11 (10%), polyester-37 (35%), coconut oil (0.5%), jojoba oil (0.25%), argan oil (0.25%), isopropyl palmitate (2%), dicaprylyl carbonate (1%), dimethyl isosorbide (0.625%), propylene glycol (1%), citric acid (QS), additional antimicrobial agent/s (QS), fragrance (QS). Combined amount of polyester-11 and polyester-37: 45%.

Example 22: water (balance), polyester-11 (45%), polyester-37 (45%), isopropyl palmitate (1%), citric acid (QS), additional antimicrobial agent/s (QS), fragrance (QS). Combined amount of polyester-11 and polyester-37: 90%.

Example 23: water (balance), polyester-11 (5%), polyester-37 (85%), isopropyl myristate (2%), citric acid (QS), additional antimicrobial agent/s (QS), fragrance (QS). Combined amount of polyester-11 and polyester-37: 90%.

Example 24: water (balance), polyester-11 (85%), polyester-37 (5%), isopropyl myristate (1%), citric acid (QS), additional antimicrobial agent/s (QS), fragrance (QS). Combined amount of polyester-11 and polyester-37: 90%.

Example 25: polyester-11 (50%), polyester-37 (50%). Combined amount of polyester-11 and polyester 37: 100%.

Add ingredients into a mixing vessel in the order shown. Mix after each ingredient addition to ensure solubility and homogeneity, before adding a subsequent ingredient.

### Lotion Compositions

Example 26: water (balance), cetrimonium chloride (1%), guar hydroxypropyltrimonium chloride (0.1%), triheptanoin (2%), bis-cetearyl amodimethicone (1%), water (and) silicone quaternium-18 (and) trideceth-6 (and) trideceth-12 (0.5%), dimethicone (0.5%), acrylates/C₁₀₋₃₀ alkyl acrylate crosspolymer (0.5%), VP/VA copolymer (1.5%), VP/methacrylamide/vinyl imidazole copolymer (10%), cetearyl alcohol (3%), cetyl alcohol (1%), polyester-11 (1%), polyester-37 (1%), glycerin (3%), alkane (1%), isopropyl palmitate (0.75%), dimethyl isosorbide (0.16%), propanediol (0.24%), citric acid (QS), additional antimicrobial agent/s (QS), fragrance (QS). Combined amount of polyester-11 and polyester-37: 2%.

Example 27: water (balance), polyquaternium-11 (2%), amidoamine (0.5%), guar hydroxypropyltrimonium chloride (0.2%), polyquaternium-37 (0.75%), triheptanoin (4%), bis-cetearyl amodimethicone (2%), water (and) silicone quaternium-18 (and) trideceth-6 (and) trideceth-12 (0.01%), dimethicone (5%), panthenol (0.25%), stearyl alcohol (2.5%), cetyl alcohol (0.5%), polyester-11 (1.5%), polyester-37 (3%), coconut oil (1%), glycerin (0.5%), isopropyl myristate (1.5%), propanediol (0.75%), isopentyldiol (0.5%), citric acid (QS), additional antimicrobial agent/s (QS), fragrance (QS). Combined amount of polyester-11 and polyester-37: 4.5%.

Example 28: water (balance), cetrimonium chloride (0.5%), amidoamine (1%), polyquaternium-37 (1%), water (and) silicone quaternium-18 (and) trideceth-6 (and) trideceth-12 (0.25%), dimethicone (0.5%), panthenol (0.1%), PVP/DMAPA acrylates copolymer (1%), stearyl alcohol (0.5%), cetearyl alcohol (3%), polyester-11 (0.2%), polyester-37 (2%), glycerin (6%), jojoba oil (0.25%), argan oil (0.25%), isopropyl palmitate (0.25%), isopentyldiol (0.5%), citric acid (QS), additional antimicrobial agent/s (QS), fragrance (QS). Combined amount of polyester-11 and polyester-37: 2.2%.

Example 29: water (balance), polyquaternium-11 (1.5%), diheptyl succinate (0.23%), capryloyl glycerin/sebacic acid copolymer (0.27%), panthenol (0.25%), acrylates/C₁₀₋₃₀ alkyl acrylate crosspolymer (0.2%), PVP/DMAPA acrylates copolymer (0.5%), polyester-11 (3%), polyester-37 (3%), coconut oil (0.5%), glycerin (2%), alkane (1%), isopropyl myristate (0.5%), propanediol (1%), citric acid (QS), additional antimicrobial agent/s (QS), fragrance (QS). Combined amount of polyester-11 and polyester-37: 6%.

Example 30: water (balance), amidoamine (0.3%), panthenol (0.5%), PVP (0.5%), PVP/DMAPA acrylates copolymer (0.25%), polyester-11 (1%), polyester-37 (0.5%), isopropyl palmitate (2%), dicaprylyl carbonate (1%), dimethyl isosorbide (0.625%), propylene glycol (1%), citric acid (QS), additional antimicrobial agent/s (QS), fragrance (QS). Combined amount of polyester-11 and polyester-37: 1.5%.

Add ingredients into a mixing vessel in the order shown. Mix after each ingredient addition to ensure solubility and homogeneity, before adding a subsequent ingredient.

### Foam Compositions

Example 31: water (balance), decyl glucoside (1%), coco-glucoside (1%), cocamidopropyl betaine (0.5%), polyester-11 (1%), polyester-37 (1%), glycerin (3%), alkane (1%), isopropyl palmitate (0.75%), butylene glycol (3%), VP/VA copolymer (1.5%), VP/methacrylamide/vinyl imidazole copolymer (10%), dimethyl isosorbide (0.5%), benzyl alcohol (0.5%), propylene glycol (1%), citric acid (QS), additional antimicrobial agent/s (QS), fragrance (QS). Combined amount of polyester-11 and polyester-37: 2%.

Example 32: water (balance), decyl glucoside (1.5%), polyester-11 (1%), polyester-37 (0.1%), coconut oil (1%), glycerin (0.5%), isopropyl myristate (1.5%), butylene glycol (5%), dimethicone (0.3%), dimethyl isosorbide (1.5%), benzyl alcohol (0.5%), citric acid (QS), additional antimicrobial agent/s (QS), fragrance (QS). Combined amount of polyester-11 and polyester-37: 1.1%.

Example 33: water (balance), decyl glucoside (2%), polyester-11 (0.2%), polyester-37 (2%), glycerin (6%), jojoba oil (0.25%), argan oil (0.25%), isopropyl palmitate (0.25%), dimethicone (0.75%), water (and) silicone quaternium-18 (and) trideceth-6 (and) trideceth-12 (0.5%), dimethyl isosorbide (1%), propylene glycol (0.5%), citric acid (QS), additional antimicrobial agent/s (QS), fragrance (QS). Combined amount of polyester-11 and polyester-37: 2.2%.

Example 34: water (balance), coco-glucoside (3%), cocamidopropyl betaine (1%), polyester-11 (0.5%), polyester-37 (0.5%), coconut oil (0.5%), glycerin (2%), alkane (1%), isopropyl myristate (0.5%), diheptyl succinate (0.23%), capryloyl glycerin/sebacic acid copolymer (0.27%), butylene glycol (1%), PVP (1.5%), VP/VA copolymer (0.5%), dimethyl isosorbide (1%), benzyl alcohol (0.5%), propylene glycol (0.5%), citric acid (QS), additional antimicrobial agent/s (QS), fragrance (QS). Combined amount of polyester-11 and polyester-37: 1%.

Example 35: water (balance), decyl glucoside (2.5%), coco-glucoside (2.5%), cocamidopropyl betaine (0.5%), polyester-11 (1%), polyester-37 (0.5%), isopropyl palmitate (2%), dicaprylyl carbonate (1%), PVP (0.5%), VP/VA copolymer (1%), VP/methacrylamide/vinyl imidazole copolymer (10%), dimethicone (0.75%), water (and) silicone quaternium-18 (and) trideceth-6 (and) trideceth-12 (0.5%), dimethyl isosorbide (2%), citric acid (QS), additional antimicrobial agent/s (QS), fragrance (QS). Combined amount of polyester-11 and polyester-37: 1.5%.

Add ingredients into a mixing vessel in the order shown. Heat to 70°C-80°C as necessary for solid waxes. Mix after each ingredient addition to ensure solubility and homogeneity, before adding a subsequent ingredient.

### Rinse Compositions

Example 36: water (balance), behentrimonium chloride (1%), cetrimonium chloride (1%), decyl glucoside (0.2%), coco-glucoside (0.2%), VP/VA copolymer (1.5%), VP/methacrylamide/vinyl imidazole copolymer (10%), dimethicone (1%), water (and) silicone quaternium-18 (and) trideceth-6 (and) trideceth-12 (0.5%), polyester-11 (1%), polyester-37 (1%), glycerin (3%), alkane (1%), isopropyl palmitate (0.75%), propylene glycol (50%), benzyl alcohol (2%), citric acid (QS), additional antimicrobial agent/s (QS), fragrance (QS). Combined amount of polyester-11 and polyester-37: 2%.

Example 37: water (balance), amidoamine (0.5%), behentrimonium chloride (0.3%), cetrimonium chloride (0.25%), decyl glucoside (1%), dimethicone (0.25%), polyester-11 (1.5%), polyester-37 (3%), coconut oil (1%), glycerin (0.5%), isopropyl myristate (1.5%), propylene glycol (30%), ethanol (15%), benzyl alcohol (0.5%), citric acid (QS), additional antimicrobial agent/s (QS), fragrance (QS). Combined amount of polyester-11 and polyester-37: 4.5%.

Example 38: water (balance), amidoamine (1%), decyl glucoside (2%), polyester-11 (0.2%), polyester-37 (2%), glycerin (6%), jojoba oil (0.25%), argan oil (0.25%), isopropyl palmitate (0.25%), propylene glycol (25%), ethanol (10%), citric acid (QS), additional antimicrobial agent/s (QS), fragrance (QS). Combined amount of polyester-11 and polyester-37: 2.2%.

Example 39: water (balance), cetrimonium chloride (3%), coco-glucoside (1%), PVP (1.5%), VP/VA copolymer (0.5%), polyester-11 (3%), polyester-37 (3%), coconut oil (0.5%), glycerin (2%), alkane (1%), isopropyl myristate (0.5%), propylene glycol (20%), diheptyl succinate (0.23%), capryloyl glycerin/sebacic acid copolymer (0.27%), ethanol (10%), benzyl alcohol (0.5%), citric acid (QS), additional antimicrobial agent/s (QS), fragrance (QS). Combined amount of polyester-11 and polyester-37: 6%.

Example 40: water (balance), behentrimonium chloride (2.5%), decyl glucoside (2.5%), coco-glucoside (2.5%), PVP (0.5%), VP/VA copolymer (1%), VP/methacrylamide/vinyl imidazole copolymer (10%), dimethicone (0.5%), water (and) silicone quaternium-18 (and) trideceth-6 (and) trideceth-12 (0.01%), polyester-11 (1%), polyester-37 (0.5%), isopropyl palmitate (2%), dicaprylyl carbonate (1%), propylene glycol (50%), ethanol (12.5%), citric acid (QS), additional antimicrobial agent/s (QS), fragrance (QS). Combined amount of polyester-11 and polyester-37: 1.5%.

Add ingredients into a mixing vessel in the order shown. Heat to 70°C-80°C as necessary for solid waxes. Mix after each ingredient addition to ensure solubility and homogeneity, before adding a subsequent ingredient.

### Methods of Use

In a further aspect, a method of using a composition as disclosed herein is disclosed. The method may comprise contacting a composition as disclosed herein with a surface of keratin, such as hair.

The following demonstrates some general, non-limiting examples of a method of use for the composition:
As a first method, a user releases the composition from its packaging onto one's hand. This may be, for instance, squeezing from a tube, pumping from a bottle, or scooping from a jar. An amount of the composition used will vary from one user to another, but can range, for instance, from 0.25g to 20g, or from 0.5g to 15g, or from 1g to 10g, or from 2g to 8g, or from 5g to 7g. The amount of the composition is then rubbed into the user's hair and allowed to remain in contact with the surface of keratin, for example, from 2 to 10 minutes. The user may then either leave the amount of the composition in (i.e., leave-on), or rinse out with water (i.e., rinse-off).

As a second method the two polyesteramines (henceforth, a booster blend) could be provided in a separate dosage meter (i.e., a unit dosage), to be combined with another composition (e.g., shampoo, conditioner, lotion that does not contain the booster blend) immediately prior to application to a surface of keratin (e.g., hair or skin) for boosting/enhancing properties of the composition such as slippage across the surface of keratin. For the purpose of this application, a "unit dosage" is defined as a pre-determined amount for usage. The following are non-limiting examples.

For instance, the booster blend could be contained within a bottle having a pump. A user would obtain the other composition (for example, squeezed from a tube into one's hand), and then dispense the booster blend onto the other composition via pumping a desired number of times (for instance, 1-4 times). That is, a unit dosage is delivered with each pump. The other composition and the booster blend would then be rubbed together and applied to hair or skin.

In another instance, the booster blend could be contained within a bottle having a dropper. The booster blend could then be applied to the other composition via a desired number of drops (for instance, 1-4 drops). That is, a unit dosage is delivered with each drop. The other composition and the booster blend would then be rubbed together and applied to hair or skin.

In a further instance, the booster blend could be contained in a pre-determined amount within a capsule. That is, the capsule contains a unit dosage. The capsule would be broken to release the booster blend over the other composition. The other composition and the booster blend would then be rubbed together and applied to hair or skin.

Furthermore, in any of the above forms, the booster blend could also be applied to wet hair immediately after washing one's hair. The booster blend could also be applied to dry hair as a touch-up during the day, or not immediately after washing one's hair.

The compositions and methods may comprise the elements of the compositions and methods as described herein, as well as any additional element described herein or otherwise useful in the disclosed compositions and methods.

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Percent ranges of amounts, and other numerical values, as disclosed herein, are understood to include the recited value +/-1% unless otherwise specified.

All references, including patent applications, patent publications, and non-patent literature, that are referred to in the present specification are incorporated by reference herein, unless it is expressly indicated that they are not incorporated by reference herein.

Having shown and described various aspects of the present invention, further adaptations of the compositions and methods described herein may be accomplished by appropriate modifications by one of ordinary skill in the art without departing from the scope of the present invention. Several of such potential modifications have been mentioned, and others will be apparent to those skilled in the art. For instance, the examples, materials, ratios, steps, and the like discussed above are illustrative and are not required. Accordingly, the scope of the present invention should be considered in terms of any claims that may be presented and is understood not to be limited to the details of composition and operation described in the specification.

## Claims

1. A composition, for application to a surface of keratin, comprising:
from 0.1% to 99.9% of a first polyesteramine as a first conditioning agent by weight of said composition; and
from 0.1% to 99.9% a second polyesteramine as a second conditioning agent by weight of said composition.

2. Said composition of claim 1, wherein: said
first polyesteramine is polyester-11; and
said second polyesteramine is polyester-37.

3. Said composition of claim 2, wherein a ratio of polyester-11 to polyester-37 ranges from 1:999 to 999:1, in particular wherein said ratio is 1:1.

4. Said composition of claim 1, wherein a combined amount of said first polyesteramine and said second polyesteramine ranges from 0.1% to 99.9% by weight of said composition, in particular wherein said combined amount of said first polyesteramine and said second polyesteramine ranges from 0.2% to 85% by weight of said composition.

5. Said composition of claim 1, further comprising at least one additional conditioning agent, in particular wherein said at least one additional conditioning agent is selected from alkanes, fatty acids, triglycerides, polyols, cationic compounds, and combinations thereof.

6. A composition, for application to a surface of keratin, comprising:
from 0.1% to 99.9% polyester-11 as a first conditioning agent by weight of said composition; and
from 0.1% to 99.9% polyester-37 as a second conditioning agent by weight of said composition.

7. Said composition of claim 6, wherein a ratio of polyester-11 to polyester-37 ranges from 1:999 to 999:1, in particular wherein said ratio is 1:1.

8. Said composition of claim 6, wherein a combined amount of polyester-11 and polyester-37 ranges from 0.1% to 99.9% by weight of said composition, in particular wherein said combined amount of polyester-11 and polyester-37 ranges from 0.2% to 85% by weight of said composition.

9. Said composition of claim 6, further comprising at least one additional conditioning agent, in particular wherein said at least one additional conditioning agent is selected from alkanes, fatty acids, triglycerides, polyols, cationic compounds, and combinations thereof.

10. A composition, for application to a surface of keratin, comprising:
from 0.1% to 99.9% polyester-11 as a first conditioning agent by weight of said composition; and
from 0.1% to 99.9% polyester-37 as a second conditioning agent by weight of said composition; wherein a ratio of polyester-11 to polyester-37 is 1:1.

11. Said composition of claim 10, wherein a combined amount of polyester-11 and polyester-37 ranges from 0.1% to 99.9% by weight of said composition, in particular wherein said combined amount of polyester-11 and polyester-37 ranges from 0.2% to 85% by weight of said composition.

12. Said composition of claim 10, further comprising an additional conditioning agent, in particular wherein said at least one additional conditioning agent is selected from alkanes, fatty acids, triglycerides, polyols, cationic compounds, and combinations thereof.
